# FASCICULE DE BREVET EUROPEEN

(11) **EP 0 399 902 B1**
(45) Date de publication et mention de la délivrance du brevet: **22.12.1993**
(21) Numéro de dépôt: 90401369.5
(22) Date de dépôt: 22.05.1990
(51) Int. Cl.: A61K 9/14, A61K 9/18, A61K 47/48

(54) **Nouvelle forme pharmaceutique poreuse et sa préparation**
Poröse Einheitsform und Verfahren
Porous pharmaceutical form and process to prepare it

(30) Priorité: 24.05.1989 FR 8906781
(43) Date de publication de la demande: 28.11.1990
(73) Titulaire: RHONE-POULENC SANTE, 92165 Antony Cédex (FR)
(72) Inventeur: Courteille, Frédéric, F-94230 Cachan (FR); Vanhoeve, Magali, F-91600 Savigny s/Orge (FR)
(74) Mandataire: Savina, Jacques

(56) Documents cités:
- EP-A- 0 197 571
- FR-A- 2 204 421

## Description

La présente invention concerne une pâte destinée à la préparation d'une nouvelle forme pharmaceutique solide et unitaire, caractérisée en ce qu'elle est lyophilisée et d'aspect homogène et poreux, ainsi que son procédé de préparation.

La demande française FR 2 204 421 décrit la stabilisation de solutions de prostaglandines, sous forme de clathrate de cyclodextrine et de prostaglandine auquel est ajouté une quantité déterminée d'acide citrique ou ascorbique.

La demande européenne EP 197 571 décrit des dérivés de γ-cyclodextrines et leur association avec divers principes actifs.

Les brevets français 2 036 890 et 2 366 835 décrivent des formes pharmaceutiques ayant pour caractéristique de se dissoudre ou de se désintégrer rapidement en milieu aqueux ou dans la salive.

Cependant, le problème du masquage du goût de médicaments destinés à l'administration orale, sans pour autant affecter la biodisponibilité du principe actif n'a jamais été résolu. Notamment en ce qui concerne l'administration de telles substances pharmaceutiques chez le jeune enfant et chez le vieillard ainsi que chez tout sujet dont la déglutition est difficile et peut poser un problème.

La nouvelle forme pharmaceutique selon l'invention, du fait de sa désagrégation ou de sa dissolution rapide en milieu aqueux ou dans la salive, rend possible l'administration chez de tels sujets tout en combinant l'avantage du masquage du goût de substances amères, irritantes, acides... ou de saveur inacceptable, qui ne pouvaient pas être facilement administrées par voie orale ainsi que l'avantage d'une dissolution contrôlée du principe actif assurant le maintien et même l'amélioration de la biodisponibilité du produit.

En outre, la nouvelle forme pharmaceutique qui présente l'avantage d'une utilisation ambulatoire, présente également l'avantage d'une mise à disposition immédiate, contrairement aux comprimés ou aux gélules dont le contenu est trop compact pour assurer une vitesse de désagrégation convenable.

La nouvelle forme pharmaceutique lyophilisée et poreuse selon l'invention contient :
a) un composé d'inclusion comportant :
   - un ou éventuellement plusieurs principes actifs,
   - une quantité prédéterminée de cyclodextrine,
   - éventuellement un additif facilitant l'inclusion ;
b) au moins une substance choisie parmi :
   - des diluants,
   - des liants ;
c) éventuellement un ou plusieurs additifs destinés à :
   - améliorer le goût,
   - améliorer le délitement,
   - modifier la couleur,
   - améliorer la conservation.

On entend par principe actif convenant pour cette nouvelle forme pharmaceutique, toute matière biologiquement active et plus spécialement toute molécule pouvant présenter des difficultés de formulation liées à des problèmes de goût, de faible solubilité ou d'insolubilité, d'instabilité et/ou de biodisponibilité.

A titre non limitatif, parmi les matières actives couramment utilisées peuvent être cités les anti-rhumatismaux et anti-inflammatoires non stéroïdiens (kétoprofène, ibuprofène, flurbipro-fène, indométacine, phénylbutazone, allopurinol...) les analgésiques opiacées ou non (paracétamol, phénacétine...), les antitussifs (codéine, codéthyline, alimémazine...), les psychotropes (trimipramine, amineptine, chlorpromazine et dérivés des phénothia-zines, diazépam, lorazepan, nitrazépam, méprobamate, zopiclone, suri-clone et dérivés de la famille des cyclopyrrolones...), les stéroïdes (hydrocortisone, cortisone, progestérone, testostérone, prednisolone, triamcinolone, dexaméthazone, betaméthazone, paraméthazone, fluocino-lone, béclométhazone...), les barbituriques (barbital, allobarbital, phénobarbital, pentobarbital, amobarbital...), les antimicrobiens (péfloxacine et dérivés de la classe des quinolones, tétracyclines, synergistines, métronidazole...) les médicaments destinés au traitement des allergies, les anti-asthmatiques, les vitamines (vitamine A, vitamine E, vitamines du groupe D, vitamine K), les antispasmodiques et antisécrétoires (oméprazole), les cardiovasculaires et les vasodilateurs cérébraux (quinacaïnol, oxprénolol, propanolol, nicergoline...), les protecteurs cérébraux, les protecteurs hépatiques, les agents thérapeutiques du tractus gastro-intestinal, les contraceptifs, les vaccins...

De plus, la nouvelle forme pharmaceutique peut permettre une présentation solide de principes actifs liquides aux conditions habituelles d'utilisation.

Il est entendu que cette nouvelle forme unitaire peut s'appliquer à l'administration de toutes sortes de substances, aussi bien en médecine humaine que vétérinaire, ainsi qu aux agents de nutrition, aux agents de diagnostic, aux agents cosmétiques, d'hygiène et de diététique (modification de l'haleine par exemple) ou même dans le cadre d'adjuvants d'alimentation.

La cyclodextrine employée peut être indifféremment choisie parmi les cyclodextrines α, β ou γ ou les cyclodextrines polymérisées ou substituées par exemple par des radicaux hydroxyéthyle ou hydroxypropyle ou les aminocyclodextrines, mais d'une manière générale on préférera employer la β-cyclodextrine.

On entend par diluant des matières pharmaceutiquement acceptables, de préférence solubles, qui améliorent les propriétés physiques de la nouvelle forme galénique. Ces substances peuvent être notamment choisies parmi le mannitol, le lactose, le glycocolle, le sorbitol, le glucose, les maltodextrines, les cyclodextrines, ou leurs mélanges, ou éventuellement parmi les oxydes (oxyde de magnésium), les carbonates (carbonate de calcium), les phosphates (phosphate tricalcique) ou les celluloses (cellulose microcristalline).

On entend par liant, toute matière soluble ou dispersible dans l'eau, acceptable du point de vue pharmaceutique et inerte vis à vis du composé d'inclusion. Ces matières sont notamment choisies parmi les polypeptides comme la gélatine ou la gélatine partiellement hydrolysée, des colloïdes, des polysaccharides à poids moléculaire élevé, des hauts polymères pouvant donner des solutions colloïdales, par exemple des gommes naturelles (gomme arabique, gomme adragante...), des gommes synthétiques ou hémisynthétiques (glycosylglucanes, gomme xanthane...), le dextrane, la dextrine, les alginates (alginate de sodium), les pectinates, les dérivés de la cellulose (cellulose microcristalline, carboxyméthylcellulose...), dérivés hydrodispersibles de l'amidon, les silices colloïdales, les bentonites, ou encore d'autres matières support telles que l'alcool polyvinylique, la polyvinylpyrrolidone, les polyéthylèneglycols (PEG 20 000, PEG 6 000 notamment), des polymères acryliques ou des copolymères, ou encore des mélanges de matières telles que citées ci-dessus.

Il est entendu que la nouvelle forme pharmaceutique doit nécessairement contenir au moins une substance choisie parmi les diluants et les liants cités ci-dessus, mais il peut être également avantageux de faire intervenir à la fois un ou plusieurs diluants et/ou un ou plusieurs liants.

En outre la nouvelle forme galénique peut contenir d'autres additifs comme des colorants, des substances modifiant le goût, des agents conservateurs, des agents destinés à empêcher le délitement, ou toute autre substance compatible avec le reste du mélange.

Les substances modifiant le goût peuvent être notamment le saccharose, le glucose, le xylose, le sorbitol, le mannitol, le xylitol, la saccharine, les saccharinates, les cyclamates, l'aspartame, ou encore les acides citrique, ascorbique ou tartrique, ou tout autre substance habituellement utilisée pour la modification du goût dans l'industrie alimentaire ou pharmaceutique et qui soit compatible avec les produits mis en présence.

Les agents colorants et agents conservateurs sont ceux qui sont habituellement utilisés dans l'industrie pharmaceutique et alimentaire.

Parmi les agents améliorant le délitement peuvent être utilisés des diluants hydrophiles ou des agents de désagrégation. Notamment des sucres (lactose, glucose, mannitol, lévulose, sorbitol maltodextrine par exemple) ou de la silice.

Les agents facilitant l'inclusion peuvent être notamment choisis parmi les solutions tampon ou autres solutions d'électrolytes ou parmi les cosolvants du principe actif. Par exemple on peut avantageusement ajouter du chlorure de sodium ou du dioctyl sulfosuccinate de sodium.

La présente invention concerne également la préparation de la nouvelle forme pharmaceutique selon les opérations suivantes :
1) préparation d'une pâte contenant les différents constituants énumérés ci-dessus en a) à c), ainsi qu'une quantité d'eau convenable de manière à ajuster la viscosité de la suspension obtenue,
2) division de la pâte en quantités unitaires de forme et de volume prédéterminés,
3) lyophilisation.

Il est entendu que le composé d'inclusion formé par le principe actif et la cyclodextrine peut être alternativement préparé préalablement à la constitution de la pâte à lyophiliser, ou in situ au cours de la préparation de la pâte. Cette seconde alternative est particulièrement avantageuse puisqu'elle permet de préparer le mélange à lyophiliser en une seule opération.

Par ailleurs, il est entendu que la division du produit peut également être effectuée mécaniquement après lyophilisation, mais il est préférable de répartir la pâte dans des alvéoles de forme et de dimensions prédéterminées, préalablement à l'opération de lyophilisation. Le dosage du (ou des) principe(s) actif(s) et, la forme et les dimensions des alvéoles étant calculées de manière à obtenir une quantité précise de principe actif dans chaque dose unitaire.

Lorsque le composé d'inclusion est préparé préalablement, il peut être obtenu par voie liquide (coprécipitation) ou par voie solide (malaxage). La quantité relative (en moles) du principe actif par rapport à la cyclodextrine varie dans des proportions allant de 1/1 à 1/10. Le composé peut être notamment préparé par mélange des quantités convenables en présence d'une faible quantité d'eau (de manière à obtenir une pâte assez fluide pour rendre possible le processus d'inclusion) puis séchage de la suspension obtenue.

Lorsque le composé d'inclusion est préparé in situ les constituants sont introduits directement dans la pâte destinée à la lyophilisation, dans des proportions identiques aux proportions définies ci-dessus.

D'une manière générale la quantité totale de composé d'inclusion est variable en fonction de la nature du principe actif, mais il est bien entendu que cette nouvelle forme solide peut permettre de préparer des doses unitaires à forte teneur en principe actif. D'une manière générale la quantité de composé d'inclusion peut aller jusqu'à 95 % en poids par rapport à la matière sèche.

Un autre avantage de la nouvelle forme galénique est de permettre de réaliser une meilleure homogénéité de mélange dans le cas de principes actifs très faiblement dosés, du fait de l'augmentation de poids moléculaire global lors de la réalisation du composé d'inclusion.

Les diluants peuvent constituer de 0 à 45 % par rapport à la masse sèche totale du lyophilisat.

D'une façon générale, les liants ne sont pas toujours indispensables, mais ils peuvent constituer de 0,01 à 10 % par rapport à la masse sèche du lyophilisat.

La quantité d'eau introduite pour constituer la pâte à lyophiliser est déterminée de telle manière que la suspension obtenue possède des caractéristiques rhéologiques permettant une bonne division du produit (écoulement, homogénéité du mélange, régularité du volume divisé, stabilité de la supension durant la division). La quantité d'eau sera le plus souvent ajustée de telle manière que la masse solide constitue environ 50 % du mélange. D'une manière générale elle pourra varier de 35 à 80 % par rapport à la masse sèche du mélange.

Le mélange constituant la pâte à lyophiliser ou le composé d'inclusion (lorsque celui-ci est préparé séparément) sont généralement préparés à température ambiante ; mais ils peuvent également être préparés à une température allant de 5°C jusqu'à environ 80°C (à condition que le principe actif concerné soit stable à cette température).

Il est entendu que la modification de la quantité de liant, de diluant ou d'eau ou de la nature des constituants peut entraîner des modifications de la qualité de la forme unitaire préparée, et permettent ainsi de préparer un produit à dissolution contrôlée.

La division de la suspension se fait manuellement ou automatiquement, dans le matériau de conditionnement primaire. D'une manière générale, les alvéoles prévues à cet effet sont constituées de chlorure de polyvinyle.

La lyophilisation donne à la nouvelle forme pharmaceutique ainsi obtenue, une structure poreuse permettant un délitement rapide dans l'eau ou directement dans la salive.

La nouvelle forme solide unitaire, selon l'invention est tout spécialement indiquée pour l'administration orale, mais elle peut être également utilisée pour l'administration par voie rectale ou vaginale.

Compte tenu des avantages qu'elle présente, la nouvelle forme unitaire est tout spécialement indiquée pour les formulations pharmaceutiques destinées à la pédiatrie ou à la gériatrie. Elle est particulièrement intéressante dans le cas de principes actifs insolubles, instables, de saveur inacceptable ou de biodisponibilité insuffisante.

Les exemples suivants illustrent la présente invention.

### Exemple 1 :

Dans un mélangeur planétaire de type OLSA, on prépare un pré-mélange contenant le principe actif et les excipients et ayant la composition suivante (formule unitaire):
- Kétoprofène 0,025 g
- β-cyclodextrine 0,554 g
- Dextrane 70 0,020 g
- Mannitol 0,100 g
- Arôme 0,030 g
- Aspartame 0,010 g

La masse pulvérulente est mélangée à sec pendant 30 minutes puis additionnée de 0,310 g d'eau par unité et malaxée pendant une heure à température ambiante.

La suspension concentrée ainsi obtenue est divisée par une diviseuse CITUS, dans des alvéoles de chlorure de polyvinyle de 1,2 cm3.

La feuille alvéolée de chlorure de polyvinyle contenant la suspension est introduite dans un lyophilisateur immédiatement après division, pour être congelée à une température de - 50°C sous pression atmosphérique, pendant environ 2 heures. Après dessication pendant 12 heures sous pression réduite, la température est remontée progressivement à 30°C par paliers de 5°C.

Les lyophilisats obtenus sont directement conditionnés par thermoscellage d'un film d'aluminium suffisamment fin pour permettre l'extrusion hors des alvéoles sans effritement.

Le temps de délitement du lyophilisat dans l'eau varie de 1 à 3 minutes.

### Exemple 2 :

En opérant comme décrit précédemment à l'exemple 1 mais à partir des constituants ci-dessous (formule unitaire):
- Kétoprofène 0,050 g
- β-cyclodextrine 1,108 g
- Dextrane 70 0,020 g
- Arôme 0,030 g
- Aspartame 0,010 g

puis addition de 0,480 g d'eau, on prépare des lyophilisats répartis dans des alvéoles de chlorure de polyvinyle de 1,6 cm³ ayant des qualités de dureté et de délitement convenables pour leur utilisation selon la présente invention.

### Exemple 3 :

En opérant comme décrit précédemment à l'exemple 1 mais à partir des constituants suivants (formule unitaire):
- Kétoprofène 0,025 g
- β-cyclodextrine 0,558 g
- Silice pulvérulée 0,004 g
- Mannitol 0,101 g
- Lactose 0,101 g
- Arôme 0,036 g
- Aspartame 0,008 g

puis addition de 0,500 g d'eau, on prépare des lyophilisats répartis dans des alvéoles de chlorure de polyvinyle de 1,6 cm³ ayant des qualités de dureté et de délitement convenable pour leur utilisation selon la présente invention.

### Exemple 4 :

En opérant comme décrit précédemment à l'exemple 1 mais à partir de :
- Kétoprofène 0,025 g
- β-cyclodextrine 0,558 g
- Mannitol 0,294 g
- Lactose 0,146 g
- Arôme 0,046 g
- Aspartame 0,011 g

On obtient après addition de 0,648 g d'eau, malaxage pendant 1 heure à température ambiante, division dans des alvéoles de chlorure de polyvinyle de 1,6 cm³ puis lyophilisation et conditionnement, un lyophilisat de bonne qualité de dureté et de délitement.

### Exemple 5 :

En opérant comme décrit précédemment à l'exemple 1, mais à partir de (formule unitaire):
- Kétoprofène 0,025 g
- β-cyclodextrine 0,557 g
- Dioctyl sulfosuccinate de sodium 0,001 g
- Lactose 0,086 g
- Sorbitol 0,029 g
- Arôme 0,032 g
- Aspartame 0,007 g

on obtient après addition de 0,470 g d'eau, malaxage pendant 1 heure à température ambiante, division dans des alvéoles de chlorure de polyvinyle de 1,2 cm³ puis lyophilisation et conditionnement, un lyophilisat de qualité convenable de dureté, et de délitement.

### Exemple 6 :

Dans un mélangeur planétaire de type OLSA on prépare un pré-mélange de (formule unitaire):
- Kétoprofène 0,025 g
- β-cyclodextrine 0,558 g

par agitation pendant 5 minutes.

Le mélange obtenu est mouillé à l'eau (faible quantité) est malaxé à 30°C pendant 2 heures 30 minutes, puis séché en étuve et tamisé sur grille de 0,5 mm.

Le composé d'inclusion ainsi obtenu est mélangé à sec pendant 30 minutes avec les excipients suivants (formule unitaire):
- Mannitol 0,294 g
- Lactose 0,146 g
- Arôme 0,046 g
- Aspartame 0,011 g

Après addition de 0,864 g d'eau et malaxage pendant une heure, la suspension est divisée dans des alvéoles de chlorure de polyvinyle de 1,6 cm³ puis lyophilisée et conditionnée comme décrit précédemment à l'exemple 1.

### Exemple 7 :

En opérant comme décrit à l'exemple 1, mais en utilisant 700 mg d'eau par unité pour la préparation de la pâte à lyophiliser, on prépare des lyophilisats dosés à 25 mg de trimipramine base ayant la composition suivante :
- Trimipramine méthanesulfonate 33,16 mg
- Dextrane 70 20,00 g
- β-cyclodextrine 1000,00 g

Les lyophilisats obtenus sont solubles, insipides et non hygroscopiques.

### Exemples 8 à 13

Le principe actif et la β cyclodextrine sont mélangés à sec pendant 15 minutes afin d'obtenir un mélange homogène.

Le mélange obtenu est mouillé avec une quantité d'eau suffisante pour obtenir, après addition des excipients complémentaires, une suspension pâteuse pouvant être divisée manuellement (viscosité convenable). La quantié maximale correspondant à la limite de remplissage des alvéoles est fixée à 1,35 g pour des alvéoles de 1,2 cm³.

Le malaxage est effectué à une vitesse moyenne, à température ambiante, pendant 3 heures (temps arbitraire). 30 minutes avant la fin du malaxage, les excipients complémentaires sont ajoutés. La suspension pâteuse est divisée dans des alvéoles de chlorure de polyvinyle de 1,2 cm³ puis lyophilisation en maintenant un pallier de congélation à -10°C pendant 1 heure.

### Exemple 8

Formule unitaire:
- Zopiclone: : 0,030 g
- β cyclodextrine: : 0,220 g
- Lactose: : 0,250 g
- Mannitol: : 0,250 g
- Arôme orange: : 0,030 g
- Aspartame: : 0,010 g

On obtient après addition de 0,500 g d'eau, malaxage, division et lyophilisation, un lyophilisat de qualité convenable de dureté et de délitement (inférieur ou égal à 3 minutes).

### Exemple 9

Formule unitaire:
- Phénobarbital: : 0,100 g
- β cyclodextrine: : 0,490 g
- Lactose: : 0,100 g
- Mannitol: : 0,100 g
- Arôme orange: : 0,030 g
- Aspartame: : 0,010 g

On obtient après addition de 0,500 g d'eau, malaxage, division et lyophilisation, un lyophilisat de qualité convenable de dureté et de délitement (inférieur ou égal à 3 minutes).

### Exemple 10

Formule unitaire:
- Vitamine A: : 0,060 g
- β cyclodextrine: : 0,148 g
- Lactose: : 0,300 g
- Mannitol: : 0,300 g
- Arôme orange: : 0,030 g
- Aspartame: : 0,010 g

On obtient après addition de 0,500 g d'eau, malaxage, division et lyophilisation, un lyophilisat de qualité convenable de dureté et de délitement (inférieur ou égal à 3 minutes).

### Exemple 11

Formule unitaire:
- Essence de citron: : 0,025 g
- β cyclodextrine: : 0,250 g
- Lactose qualité lyoc: : 0,050 g
- Mannitol: : 0,050 g
- Aspartame: : 0,005 g

On obtient après addition de 0,600 g d'eau, malaxage, division et lyophilisation, un lyophilisat de qualité convenable de dureté et de délitement (inférieur ou égal à 3 minutes).

### Exemple 12

Formule unitaire:
- Pristinamycine naturelle: : 0,100 g
- β cyclodextrine: : 0,490 g
- Lactose: : 0,100 g
- Mannitol: : 0,100 g
- Arôme orange: : 0,030 g
- Aspartame: : 0,010 g

On obtient après addition de 0,750 g d'eau, malaxage, division et lyophilisation, un lyophilisat de qualité convenable de dureté et de délitement (inférieur ou égal à 3 minutes).

### Exemple 13

Formule unitaire:
- Vitamine D3: : 0,005 g
- β cyclodextrine: : 0,148 g
- Lactose qualité lyoc: : 0,300 g
- Mannitol: : 0,300 g
- Arôme: : 0,030 g
- Aspartame: : 0,010 g

On obtient après addition de 0,300 g d'eau, malaxage, division et lyophilisation, un lyophilisat de qualité convenable de dureté et de délitement (inférieur ou égal à 3 minutes).

## Revendications (Revendications pour l'(les) Etat(s) contractant(s) suivant(s): AT, BE, CH, DE, DK, FR, GB, IT, LI, LU, NL, SE)

1. Pâte destinée à la préparation par lyophylisation d'une forme pharmaceutique solide et unitaire, d'aspect homogène et poreux consistant en :
a) un composé d'inclusion pouvant aller jusqu'à 95 % en poids par rapport à la matière sèche et comportant :
- un ou éventuellement plusieurs principes actifs et,
- une quantité prédéterminée de cyclodextrine choisie parmi les cyclodextrines α, β ou γ, les cyclodextrines polymérisées ou les cyclodextrines substituées, telle que la quantité molaire relative du principe actif par rapport à la cyclodextrine soit comprise entre 1/1 et 1/10
- éventuellement un additif facilitant l'inclusion;
b) au moins une substance choisie parmi :
- des diluants pouvant constituer de 0 à 45 % par rapport à la masse sèche totale du lyophilisat,
- des liants pouvant constituer de 0,01 à 10 % par rapport à la masse sèche du lyophilisat,
les diluants étant choisis parmi le mannitol, le lactose, le glycocolle, le sorbitol, le glucose, les maltodextrines, ou leurs mélanges, ou parmi les oxydes, les carbonates, les phosphates ou les celluloses,
les liants étant choisis parmi les polypeptides, les colloïdes, les polysaccharides à poids moléculaire élevé, les hauts polymères pouvant donner des solutions colloïales, le dextrane, la dextrine, les alginates (alginate de sodium), les pectinates, les dérivés de la cellulose, les dérivés hydrodispersibles de l'amidon, les silices colloïdales, les bentonites, l'alcool polyvinylique, la polyvinylpyrrolidone, les polyéthylèneglycols, les polymères acryliques ou des copolymères, ou des mélanges de ces matières,
c) éventuellement un ou plusieurs additifs destinés à :
- améliorer le goût,
- améliorer le délitement,
- modifier la couleur,
- améliorer la conservation.
d) une quantité de 35 à 80% d'eau par rapport à la masse sèche du mélange, convenable de manière à ajuster la viscosité de la suspension obtenue pour que celle-ci possède des caractéristiques rhéologiques permettant une bonne division du produit.

2. Pâte, selon la revendication 1, caractérisée en ce que le principe actif est choisi parmi la classe des psychotropes, des analgésiques, des anti-inflammatoires ou des vitamines.

3. Pâte selon la revendication 1, caractérisée en ce que le principe actif est le kétoprofène.

4. Pâte selon la revendication 1, caractérisée en ce que le principe actif est la zopiclone.

5. Pâte selon la revendication 1, caractérisée en ce que le principe actif est la trimipramine.

6. Pâte selon la revendication 1, caractérisée en ce que le principe actif est le phénobarbital.

7. Pâte selon la revendication 1, caractérisée en ce que le principe actif est la vitamine A.

8. Procédé de préparation d'une forme pharmaceutique unitaire et solide, d'aspect homogène et poreux caractérisé en ce que l'on effectue les opérations suivantes:
1) préparation d'une pâte selon la revendication 1,
2) division de la pâte en quantités unitaires de forme et de volume prédéterminés,
3) lyophilisation,
étant entendu que les opérations 2) et 3) peuvent être effectuées indifféremment dans n'importe que ordre.

9. Procédé selon la revendication 8, caractérisé en ce que le composé d'inclusion est préparé séparément avant la préparation du mélange à lyophiliser.

10. Procédé selon la revendication 8, caractérisé en ce que le composé d'inclusion est préparé in situ pendant l'opération de préparation de la pâte à lyophiliser.

11. Utilisation d'une pâte selon la revendication 1 pour la préparation selon le procédé de la revendication 8, de compositions destinées à la médecine humaine ou vétérinaire.

12. Utilisation d'une pâte selon la revendication 1 pour la préparation selon le procédé de la revendication 8, de compositions destinées aux agents de diagnostic, à l'hygiène, la diététique, la cosmétologie ou à l'alimentation animale.

## Revendications (Revendications pour l'(les) Etat(s) contractant(s) suivant(s): ES, GR)

1. Procédé de préparation d'une forme pharmaceutique solide et unitaire, d'aspect homogène et poreux par lyophilisation d'une pâte consistant en :
a) un composé d'inclusion pouvant aller jusqu'à 95 % en poids par rapport à la matière sèche et comportant :
- un ou éventuellement plusieurs principes actifs et,
- une quantité prédéterminée de cyclodextrine choisie parmi les cyclodextrines α, β ou γ, les cyclodextrines polymérisées ou les cyclodextrines substituées, telle que la quantité molaire relative du principe actif par rapport à la cyclodextrine soit comprise entre 1/1 et 1/10
- éventuellement un additif facilitant l'inclusion;
b) au moins une substance choisie parmi :
- des diluants pouvant constituer de 0 à 45 % par rapport à la masse sèche totale du lyophilisat,
- des liants pouvant constituer de 0,01 à 10 % par rapport à la masse sèche du lyophilisat,
les diluants étant choisis parmi le mannitol, le lactose, le glycocolle, le sorbitol, le glucose, les maltodextrines, ou leurs mélanges, ou parmi les oxydes, les carbonates, les phosphates ou les celluloses,
les liants étant choisis parmi les polypeptides, les colloïdes, les polysaccharides à poids moléculaire élevé, les hauts polymères pouvant donner des solutions colloïales, le dextrane, la dextrine, les alginates (alginate de sodium), les pectinates, les dérivés de la cellulose, les dérivés hydrodispersibles de l'amidon, les silices colloïdales, les bentonites, l'alcool polyvinylique, la polyvinylpyrrolidone, les polyéthylèneglycols, les polymères acryliques ou des copolymères, ou des mélanges de ces matières,
c) éventuellement un ou plusieurs additifs destinés à :
- améliorer le goût,
- améliorer le délitement,
- modifier la couleur,
- améliorer la conservation,
ainsi qu'une quantité 35 à 80 % d'eau par rapport à la masse sèche du mélange convenable de manière à ajuster la viscosité de la suspension obtenue pour que celle-ci possède des caractéristiques rhéologiques permettant une bonne division du produit,
2) division de la pâte en quantités unitaires de forme et de volume prédéterminés,
3) lyophilisation,
étant entendu que les opérations 2) et 3) peuvent être effectuées indifféremment dans n'importe quel ordre.

2. Procédé selon la revendication 1, caractérisé en ce que le composé d'inclusion est préparé séparément avant la préparation du mélange à lyophiliser.

3. Procédé selon la revendication 1, caractérisé en ce que le composé d'inclusion est préparé in situ pendant l'opération de préparation de la pâte à lyophiliser.

## Patentansprüche (Patentansprüche für folgende(n) Vertragsstaat(en): AT, BE, CH, DE, DK, FR, GB, IT, LI, LU, NL, SE)

1. Paste für die Herstellung einer festen pharmazeutischen Einheitsform mit homogenem und porösem Erscheinungsbild, durch Lyophilisation, bestehend aus
a) einer Einschlußverbindung, die bis zu 95 Gew.-% im Verhältnis zur Trockenmasse betragen kann und umfaßt:
- einen oder gegebenenfalls mehrere Wirkstoffe, und
- eine solche vorbestimmte Menge an Cyclodextrin, ausgewählt unter den α-,β- oder γ-Cyclodextrinen, den polymerisierten Cyclodextrinen oder den substituierten Cyclodextrinen, daß die relative Molmasse des Wirkstoffes, bezogen auf das Cyclodextrin zwischen 1/1 und 1/10 liegt.
- gegebenenfalls einen Zusatzstoff. der den Einschluß erleichtert;
b) mindestens eine Substanz, ausgewählt unter:
- Verdünnungsmitteln, die 0 bis 45 % in bezug auf die Gesamt-Trockenmasse des Lyophilisates bilden können,
- Bindemitteln, die 0.01 bis 10 % in bezug auf die Trockenmasse des Lyophilisates bilden können,
wobei die Verdünnungsmittel ausgewählt werden unter Mannitol, Laktose, Glykokoll. Sorbitol, Glucose, den Maltodextrinen, oder ihren Mischungen, oder unter den Oxiden, den Carbonaten, den Phosphaten oder den Cellulosen, und
wobei die Bindemittel ausgewählt werden unter den Polypeptiden. den Kolloiden, den Polysacchariden mit hohem Molekulargewicht. den Hochpolymeren, die kolloidale Lösungen ergeben können, Dextran, Dextrin, den Alginaten (Natriumalginat), den Pectinaten, den Cellulose-Derivaten, den wasserdispergierbaren Stärke-Derivaten, den kolloidalen Kieselerden, den Bentoniten, Polyvinylalkohol, Polyvinylpyrrolidon, den Polyethylenglycolen. den Acrylpolymeren oder -copolymeren, oder den Mischungen dieser Stoffe,
c) gegebenenfalls einen oder mehrere Zusatzstoffe, die vorgesehen sind für:
- die Verbesserung des Geschmackes,
- die Verbesserung des Zerfalls,
- die Modifizierung der Farbe,
- die Verbesserung der Konservierung,
d) eine Menge von 35 bis 80 % Wasser, in bezug auf die Trockenmasse der Mischung, die geeignet ist, die Viskosität der erhaltenen Suspension in der Weise einzustellen, daß sie die rheologischen Charakteristiken besitzt, die eine gute Verteilung des Produktes ermöglichen.

2. Paste nach Anspruch 1, dadurch gekennzeichnet, daß der Wirkstoff ausgewählt wird aus der Klasse der Psychotropika, der Analgetika, der anti-inflammatorischen Mittel oder der Vitamine.

3. Paste nach Anspruch 1, dadurch gekennzeichnet, daß der Wirkstoff Ketoprofen ist.

4. Paste nach Anspruch 1, dadurch gekennzeichnet, daß der Wirkstoff Zopiclon ist.

5. Paste nach Anspruch 1, dadurch gekennzeichnet, daß der Wirkstoff Trimipramin ist.

6. Paste nach Anspruch 1, dadurch gekennzeichnet, daß der Wirkstoff Phenobarbital ist.

7. Paste nach Anspruch 1, dadurch gekennzeichnet, daß der Wirkstoff Vitamin A ist.

8. Verfahren zur Herstellung einer festen pharmazeutischen Einheitsform mit homogenem und porösem Erscheinungsbild, dadurch gekennzeichnet. daß man die folgenden Arbeitsschritte durchführt:
1) Herstellung einer Paste gemäß Anspruch 1,
2) Aufteilung der Paste in Einheitsmengen mit vorbestimmter Form und vorbestimmtem Volumen,
3) Lyophilisation,
mit der Maßgabe, daß die Arbeitsschritte 2) und 3) unabhängig von irgendeiner Reihenfolge durchgeführt werden können.

9. Verfahren nach Anspruch 8, dadurch gekennzeichnet, daß die Einschlußverbindung vor der Herstellung der zu lyophilisierenden Mischung getrennt hergestellt wird.

10. Verfahren nach Anspruch 8, dadurch gekennzeichnet, daß die Einschlußverbindung während des Arbeitsganges der Herstellung der zu lyophilisierenden Paste in situ hergestellt wird.

11. Verwendung einer Paste nach Anspruch 1 zur Herstellung von Zusammensetzungen gemäß dem Verfahren von Anspruch 8, die für die Human- oder Veterinärmedizin bestimmt sind.

12. Verwendung einer Paste nach Anspruch 1 zur Herstellung von Zusammensetzungen gemäß dem Verfahren von Anspruch 8. die für Diagnostika, Hygieneprodukte, diätetische Mittel, kosmetische Mittel oder für die Tierernährung bestimmt sind.

## Patentansprüche (Patentansprüche für folgende(n) Vertragsstaat(en): ES, GR)

1. Verfahren zur Herstellung einer festen pharmazeutischen Einheitsform mit homogenem und porösem Erscheinungsbild, durch Lyophilisation einer Paste, bestehend aus
a) einer Einschlußverbindung, die bis zu 95 Gew.-% im Verhältnis zur Trockenmasse betragen kann und umfaßt:
- einen oder gegebenenfalls mehrere Wirkstoffe, und
- eine solche vorbestimmte Menge an Cyclodextrin, ausgewählt unter den α-,β- oder γ-Cyclodextrinen, den polymerisierten Cyclodextrinen oder den substituierten Cyclodextrinen, daß die relative Molmasse des Wirkstoffes, bezogen auf das Cyclodextrin zwischen 1/1 und 1/10 liegt.
- gegebenenfalls einen Zusatzstoff, der den Einschluß erleichtert;
b) mindestens eine Substanz, ausgewählt unter:
- Verdünnungsmitteln, die 0 bis 45 % in bezug auf die Gesamt-Trockenmasse des Lyophilisates bilden können,
- Bindemitteln, die 0,01 bis 10 % in bezug auf die Trockenmasse des Lyophilisates bilden können,
wobei die Verdünnungsmittel ausgewählt werden unter Mannitol, Laktose, Glykokoll, Sorbitol, Glucose, den Maltodextrinen, oder ihren Mischungen, oder unter den Oxiden, den Carbonaten, den Phosphaten oder den Cellulosen, und
wobei die Bindemittel ausgewählt werden unter den Polypeptiden, den Kolloiden, den Polysacchariden mit hohem Molekulargewicht, den Hochpolymeren, die kolloidale Lösungen ergeben können, Dextran, Dextrin, den Alginaten (Natriumalginat), den Pectinaten, den Cellulose-Derivaten, den wasserdispergierbaren Stärke-Derivaten, den kolloidalen Kieselerden, den Bentoniten, Polyvinylalkohol, Polyvinylpyrrolidon, den Polyethylenglycolen, den Acrylpolymeren oder -copolymeren, oder den Mischungen dieser Stoffe,
c) gegebenenfalls einen oder mehrere Zusatzstoffe, die vorgesehen sind für:
- die Verbesserung des Geschmackes,
- die Verbesserung des Zerfalls,
- die Modifizierung der Farbe,
- die Verbesserung der Konservierung,
sowie einer Menge von 35 bis 80 % Wasser, in bezug auf die Trockenmasse der Mischung. die geeignet ist, die Viskosität der erhaltenen Suspension in der Weise einzustellen, daß sie die rheologischen Charakteristiken besitzt, die eine gute Verteilung des Produktes ermöglichen,
2) Aufteilung der Paste in Einheitsmengen mit vorbestimmter Form und vorbestimmtem Volumen.
3) Lyophilisation,
mit der Maßgabe, daß die Arbeitsschritte 2) und 3) unabhängig von irgendeiner Reihenfolge durchgeführt werden können.

2. Verfahren nach Anspruch 1, dadurch gekennzeichnet, daß die Einschlußverbindung vor der Herstellung der zu lyophilisierenden Mischung getrennt hergestellt wird.

3. Verfahren nach Anspruch 1, dadurch gekennzeichnet, daß die Einschlußverbindung während des Arbeitsganges der Herstellung der zu lyophilisierenden Paste in situ hergestellt wird.

## Claims (Claims for the following Contracting State(s): AT, BE, CH, DE, DK, FR, GB, IT, LI, LU, NL,SE)

1. Paste intended for the preparation by lyophilisation of a single-dose, solid pharmaceutical dosage form of homogeneous and porous appearance, consisting of:
a) an inclusion compound which can range up to 95 % by weight relative to the dry matter and containing:
- one or possibly several active principles, and
- a predetermined quantity of cyclodextrin chosen from α-, β- or γ-cyclodextrins, polymerised cyclodextrins or substituted cyclodextrins, such that the relative molar quantity of the active principle relative to the cyclodextrin is between 1:1 and 1:10,
- where appropriate, an additive facilitating inclusion;
b) at least one substance chosen from:
- diluents which can constitute from 0 to 45% relative to the total dry mass of the lyophilisate,
- binding agents which can constitute from 0.01 to 10% relative to the dry mass of the lyophilisate,
the diluents being chosen from mannitol, lactose, glycine, sorbitol, glucose, maltodextrins, or mixtures thereof, or from oxides, carbonates, phosphates or celluloses,
the binding agents being chosen from polypeptides, colloids, high molecular weight polysaccharides, high polymers capable of giving colloidal solutions, dextran, dextrin, alginates (sodium alginate), pectinates, cellulose derivatives, water-dispersible starch derivatives, colloidal silicas, bentonites, polyvinyl alcohol, polyvinylpyrrolidone, polyethylene glycols, copolymers or acrylic polymers, or mixtures of these substances;
c) if appropriate, one or more additives intended for:
- improving the taste,
- improving the disintegration,
- modifying the colour,
- improving the preservation;
d) a quantity of 35 to 50% of water, relative to the dry mass of the mixture, which is appropriate for adjusting the viscosity of the suspension obtained so that the latter possesses rheological properties permitting good division of the product.

2. Paste according to claim 1, characterised in that the active principle is chosen from the class of psychotropic drugs, analgesics, anti-inflammatories or vitamins.

3. Paste according to claim 1, characterised in that the active principle is ketoprofen.

4. Paste according to claim 1, characterised in that the active principle is zopiclone.

5. Paste according to claim 1, characterised in that the active principle is trimipramine.

6. Paste according to claim 1, characterised in that the active principle is phenobarbital.

7. Paste according to claim 1, characterised in that the active principle is vitamin A.

8. Process for preparing a single-dose, solid pharmaceutical dosage form of homogeneous and porous appearance, characterised in that the following operations are performed:
1) preparation of a paste according to claim 1,
2) division of the paste into single-dose quantities of predetermined shape and volume,
3) lyophilisation,
on the understanding that operations 2) and 3) may be performed equally well in either order.

9. Process according to claim 8, characterised in that the inclusion compound is prepared separately before the preparation of the mixture to be lyophilised.

10. Process according to claim 8, characterised in that the inclusion compound is prepared in situ during the operation of preparation of the paste to be lyophilised.

11. Use of a paste according to claim 1 for the preparation, according to the process of claim 8, of compositions intended for human or veterinary medicine.

12. Use of a paste according to claim 1 for the preparation, according to the process of claim 8, of compositions intended as diagnostic agents and for hygiene, dietetics, cosmetology or animal feeds.

## Claims (Claims for the following Contracting State(s): ES, GR)

1. Process for preparing a single-dose, solid pharmaceutical dosage form of homogeneous and porous appearance, by lyophilisation of a paste consisting of:
a) an inclusion compound which can range up to 95 % by weight relative to the dry matter and containing:
- one or possibly several active principles, and
- a predetermined quantity of cyclodextrin chosen from α-, β- or γ-cyclodextrins, polymerised cyclodextrins or substituted cyclodextrins, such that the relative molar quantity of the active principle relative to the cyclodextrin is between 1:1 and 1:10,
- where appropriate, an additive facilitating inclusion;
b) at least one substance chosen from:
- diluents which can constitute from 0 to 45% relative to the total dry mass of the lyophilisate,
- binding agents which can constitute from 0.01 to 10% relative to the dry mass of the lyophilisate,
the diluents being chosen from mannitol, lactose, glycine, sorbitol, glucose, maltodextrins, or mixtures thereof, or from oxides, carbonates, phosphates or celluloses,
the binding agents being chosen from polypeptides, colloids, high molecular weight polysaccharides, high polymers capable of giving colloidal solutions, dextran, dextrin, alginates (sodium alginate), pectinates, cellulose derivatives, water-dispersible starch derivatives, colloidal silicas, bentonites, polyvinyl alcohol, polyvinylpyrrolidone, polyethylene glycols, copolymers or acrylic polymers, or mixtures of these substances;
c) if appropriate, one or more additives intended for:
- improving the taste,
- improving the disintegration,
- modifying the colour,
- improving the preservation;
as well as a quantity of 35 to 80% of water, relative to the dry mass of the mixture, which is appropriate for adjusting the viscosity of the suspension obtained so that the latter possesses rheological characteristics permitting good division of the product,
2) division of the paste into single-dose quantities of predetermined shape and volume,
3) lyophilisation,
on the understanding that operations 2) and 3) may be performed equally well in either order.

2. Process according to claim 1, characterised in that the inclusion compound is prepared separately before the preparation of the mixture to be lyophilised.

3. Process according to claim 1, characterised in that the inclusion compound is prepared in situ during the operation of preparation of the paste to be lyophilised.
